(19) Europäisches Patentamt
European Patent Office
Office européen des brevets

(11) Publication number: **0 135 172**
**A2**

# (12) EUROPEAN PATENT APPLICATION

(21) Application number: 84109990.6

(22) Date of filing: 22.08.84

(51) Int. Cl.[4]: A 61 K 31/35
//C07D311/36

(30) Priority: 24.08.83 JP 155603/83

(43) Date of publication of application:
27.03.85 Bulletin 85/13

(84) Designated Contracting States:
BE CH DE FR GB LI NL SE

(71) Applicant: Takeda Chemical Industries, Ltd.
27, Doshomachi 2-chome Higashi-ku
Osaka-shi Osaka, 541(JP)

(72) Inventor: Yamazaki, Iwao
9-21, Hibarigaokayamate 1-chome
Takarazuka Hyogo 665(JP)

(72) Inventor: Sawa, Yoichi
19-11, Sengokuhigashi-machi
Kadoma Osaka 571(JP)

(74) Representative: von Kreisler, Alek, Dipl.-Chem. et al,
Deichmannhaus am Hauptbahnhof
D-5000 Köln 1(DE)

## (54) Method for treatment of osteoporosis.

(57) A compound of the formula

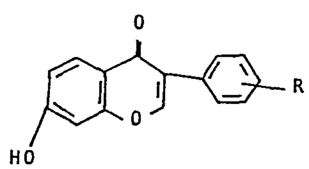

wherein R is a hydrogen atom or a hydroxy group is effective for prevention or treatment of osteoporosis.

EP 0 135 172 A2

Method for treatment of osteoporosis

This invention relates to a therapeutic means for treatment of osteoporosis.

More particularly, this invention relates to a medicament for prevention or treatment of osteoporosis, which contains a compound of the formula

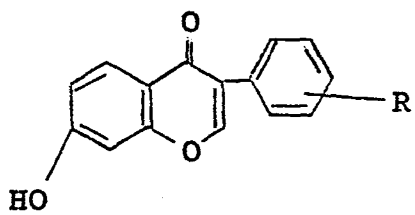

wherein R is a hydrogen atom or a hydroxy group.

Osteoporosis is a disease condition or illness which occurs frequently in postmenopausal females, particularly those in their sixties, and wherein the quantitative loss of bones progresses beyond a certain limit to thereby present some symptoms or risk manifestations. Among its main clinical manifestations are kyphosis, low back pain, and fractures of femoral neck, lower end of the radius, ribs, upper end of the humerus, etc. While the causative factors are variegated, including endocrine disorder and nutritional disorder, apparently the most important cause is a descreased secretion of estrogen due to hypoovarianism in females during the postmenopausal period. Therefore, of all the therapeutic agents for osteoporosis, the theoretically most effective drugs are estrogen preparations.

However, the estrogens so far available are so strong in effect as to cause side effects such as genital bleeding, mastodynia, hepatic disorder, etc. and, for this reason, have not been used recently on as many occasions as in the past. There are other types of therapeutic agents such as calcitonin, vitamin D and calcium preparations, which however are disadvantageous in that they are either only indefinitely effective or ineffective when administered by the oral route.

The present inventors have found that the compound of the formula (I) exhibits a milder estrogen activity than the conventional estrogens in the oral regimen and does not cause side effects which are produced by these known drugs but cures osteoporosis by stimulating secretion of calcitonin from the thyroid.

The compounds of the formula (I) which are employed in accordance with the present invention are invariably crystalline compounds which are white to pale yellowish brown in color, and are freely soluble in dimethylformamide and chloroform, soluble in ethanol and acetone and practically insoluble in water. When R in the formula (I) is a hydroxy group, it may be present in any position of the phenyl ring.

These compounds can be produced, for example, by cyclizing a 2,4-dihydroxy-phenyl (with or without a hydroxy group on the benzene ring) benzyl ketone to a benzopyran compound, and some of these compounds are known to have capillary vessel stabilizing activity (French Pharmaceutical Patent No. 1065), therapeutic effective for vascular disorders, inflammatory and vitamin-P deficiency disorders (United States Patent No. 3,352,754) or anticonvulsant activity (Japanese Patent Publication No. 32074/1972), but it has not been known that any of the compounds is useful for the treatment of osteoporosis.

As will be apparent from Test Example 5 which appears hereinafter, all of the compounds of the formula (I) are

sparingly toxic. Thus, in the studies in which the compounds were administered orally or subcutaneously to mice or rats at the technically feasible highest doses (5,000 to 10,000 mg/kg), there occurred no death nor toxic symptoms attributable to the compounds.

On the other hand, Test Examples 1 and 2 presented hereinafter show that 7-hydroxy-isoflavone [hereinafter referred to briefly as compound (I)] and 7,4'-dihydroxy-isoflavone [briefly, compound (II)], which are representative species of the compound represented by the formula (I), have mild estrogenic activity which is suited for the treatment of osteoporosis.

Test Example 1

Estrogenic activity of 7-hydroxy-isoflavone in young oophorectomized rats

Sprague-Dawley rats, 33 days old and 11 days after oophorectomy for elimination of endogenous estrogenic activity, were used in groups of 6 to 7 animals. Compound (I) was suspended in a 1% aqueous solution of hydroxypropylcellulose and administered orally for 3 days, while as a representative example of the conventional estrogen drug, estrone was dissolved in sesame oil and administered subcutaneously for 3 days. On the fourth day, each animal was autopsied and its uterine wet weight was recorded. As shown in Table 1, compound (I) at the daily dose levels of 200 mg/kg and 400 mg/kg produced uterine weight increasing effect with a dose-response curve of moderate gradient. In contrast, estrone showed uterine weight increasing effect with a dose-response curve of steep gradient.

Table 1

| Compound | Daily dose (mg/kg) | No. of animals | Uterine wet weight (mg ± S.D.) |
|---|---|---|---|
| Compound (I) | 0 (control group) | 7 | 35.0 ± 1.0 |
| | 6.25 | 7 | 32.8 ± 1.1 |
| | 12.5 | 7 | 33.4 ± 0.9 |
| | 25 | 7 | 35.1 ± 0.8 |
| | 50 | 7 | 35.3 ± 1.7 |
| | 100 | 7 | 35.9 ± 1.0 |
| | 200 | 7 | 57.9 ± 1.0* |
| | 400 | 6 | 70.4 ± 6.7* |
| Estrone | 0.0025 | 7 | 101.7 ± 4.6* |
| | 0.005 | 7 | 159.8 ± 9.4* |
| | 0.01 | 7 | 223.3 ± 12.5* |

*: Significant as compared with control group (P<0.01)

Test Example 2

Estrogenic activity of 7,4'-dihydroxy-isoflavone in young oophorectomized rats

Sprague-Dawley rats, 33 days old and 11 days after oophorectomy for elimination of endogenous estrogenic activity, were used in groups of 7 animals. Compound (II) was suspended in a 1% aqueous solution of hydroxypropylcellulose and administered orally. As shown in Table 2, compound (II) at the dose level of 400 mg/kg showed mild uterine weight increasing activity.

Table 2

| Daily dose of compound (II) (mg/kg) | No. of animals | Uterine wet weight (mg ± S.D.) |
|---|---|---|
| 0 (control group) | 7 | 31.1 ± 1.1 |
| 6.25 | 7 | 33.2 ± 0.8 |
| 25 | 7 | 32.8 ± 1.0 |
| 100 | 7 | 35.3 ± 1.3 |
| 400 | 7 | 62.3 ± 6.0* |

*: Significant as compared with control group (P<0.01)

The following Test Examples 3 and 4 show that the compounds of this invention have bone resorption-inhibiting activity which is effective for the treatment of osteoporosis.

Test Example 3

Bone resorption inhibiting activity of 7-hydroxy-isoflavone and 7,4'-dihydroxy-isoflavone in rat fetal long bone culture.

Determination of bone resorption was performed by the method of Raisz [J. Clin. Invest. 44, 103-116 (1965)]. Thus, a Sprague-Dawley rat on the 19th day of pregnancy was subcutaneously injected with 50 μ Ci of $^{45}Ca$ (isotope of calcium, $CaCl_2$ solution), and was laparotomized on the following day. The embryos were aseptically taken out, the forelimbs (radius and ulna) were cut off from the trunk under a binocular dissecting microscope, and the connective tissue and cartilage were removed as much as possible to prepare bone samples. Each bone sample was preincubated at 37°C for 24 hours in 0.6 mℓ of the medium containing 2 mg/mℓ of bovine serum albumin in $BGJ_b$ medium (Fitton-Jackson modification) [GIBCO Laboratories, Grand Island, NY 14072 U.S.A.]. Then, the sample was further incubated for 3 days in the same medium as above in which 10 μg/mℓ or 25 μg/mℓ of compound (I) or 10 μg/mℓ of compound (II) had been incorporated. Then, the radioactivity of $^{45}Ca$ in the medium and that of $^{45}Ca$ in the bone were measured and the percentage (%) of

$^{45}Ca$ released from the bone into the medium was calculated by the following formula.

Percentage (%) of $^{45}Ca$ released from bone into medium

$$= \frac{\text{Count of } ^{45}\text{Ca in medium}}{\text{Count of } ^{45}\text{Ca in medium} + \text{Count of } ^{45}\text{Ca in bone}} \times 100$$

As control, the bones of the embryos from the same litter were similarly incubated in the absence of compound (I) or (II) for 3 days. The mean ± standard deviation for the six bones per group are shown in Table 3. It is apparent that compounds (I) and (II) suppressed bone resorption.

Table 3

| | Concentration of compound | $^{45}Ca$ (%) released | |
|---|---|---|---|
| Control group | 0 | 20.6 ± 3.8 | 19.9 ± 5.0 |
| Test group 1 | Compound (I) 10 μg/mℓ | 16.5 ± 2.5* | |
| Test group 2 | Compound (I) 25 μg/mℓ | 13.5 ± 2.5* | |
| Test group 3 | Compound (II) 10 μg/mℓ | | 15.9 ± 1.3** |

* : A significant difference from the control group ($P<0.001$)

**: A significant difference from the control group ($P<0.002$)

Test Example 4

Inhibiting activity of 7,4'-dihydroxy-isoflavone to the bone resorption potentiating action of parathyroid hormone in rat fetal long bone culture.

The bone samples prepared in the same manner as Test Example 3 were pre-incubated for 24 hours in the same medium as that prepared in Test Example 3 which contains bovine serum album in $BGJ_b$ medium (Fitton-Jackson modification). Then, in the concomitant presence of PTH (parathyroid hormone, a bone resorption stimulant) and compound (II), the samples

were further incubated for 3 days and the percentage of $^{45}Ca$ released into the medium was calculated by means of the same formula as that in Test Example 3. The results are shown in Table 4. As control experiments, the same determination was made for a control group using the medium supplemented with PTH alone. It is apparent from Table 4 that compound (II) suppressed PTH-stimulated bone resorption.

Table 4

| | Concentration of compound (II) | $^{45}Ca$ (%) released |
|---|---|---|
| Control group | 0 | 30.8±4.3 |
| Test group | 10 μg/mℓ | 23.5±3.4* |

*: A significant difference from the control group (P<0.01)

Test Example 5

Acute toxicity

Five-week-old ICR mice and 5-week-old Sprague-Dawley rats were respectively used in groups of 10 males and 10 females, and suspensions of compound (I) or compound (II) in olive oil were administered orally [2,500, 5,000 and 10,000 mg/kg of each compound] or subcutaneously [1,250, 2,500 and 5,000 mg/kg]. The animals were kept under observation for 14 days. None of the groups showed deaths nor toxic symptoms attributable to compound (I) or (II) and, therefore, $LD_{50}$ values could not be calculated.

The daily dosage of the compound of the formula (I) according to this invention for human beings is generally about 1 to 50 mg/kg and preferably about 5 to 20 mg/kg for oral administration, and about 200 to 600 mg can be orally taken daily, once a day or, if necessary, in 2 to 3 divided doses. The compounds are preferably formulated into such dosage forms as tablets, capsules, etc. by the established pharmaceutical procedure. Such tablets and capsules can

be prepared using suitable excipients such as lactose, starch, etc., binders such as hydroxypropylcellulose, and lubricants such as magnesium stearate. The tablets may be sugar-coated, if necessary.

The following preparation examples are given to illustrate the invention in further detail and should not be construed as limiting the scope of the invention.

Example 1 Tablets

| | | |
|---|---|---|
| I) | 7-Hydroxy-isoflavone | 200 g |
| II) | Lactose | 15 g |
| III) | Starch | 44 g |
| IV) | Carboxymethylcellulose | 10 g |
| V) | Magnesium stearate | 1 g |

The above components I) through V) were admixed to prepare 1000 uncoated tablets with a diameter of 8.5 mm.

Example 2 Capsules

| | | |
|---|---|---|
| I) | 7,4'-Dihydroxy-isoflavone | 200 g |
| II) | Lactose | 40 g |
| III) | Starch | 50 g |
| IV) | Hydroxypropylcellulose | 7 g |
| V) | Magnesium stearate | 3 g |

The above components I) through V) were admixed and filled into 1000 No. 1 capsules.

What is claimed is:

1. A compound of the formula

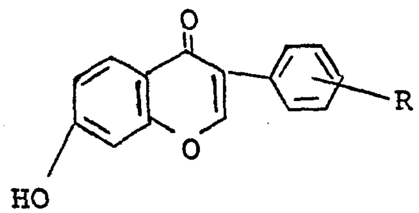

wherein R is a hydrogen atom or a hydroxy group for use in prevention or treatment of osteoporosis.

2. A pharmaceutical composition for prevention or treatment of osteoporosis, which contains an effective amount of a compound of the formula

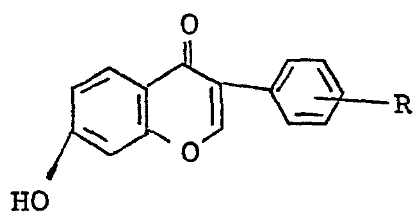

wherein R is a hydrogen atom or a hydroxy group and a pharmaceutical acceptable carrier, vehicle, lubricant or diluent therefor.

3. A pharmaceutical composition according to claim 2, which is in the form of tablet, capsule, granule, fine granule, powder or syrup.

4 A pharmaceutical composition according to claim 2, wherein the osteoporosis is that caused by decreasing secretion of estrogen due to hypoovarianism.